(19) Europäisches Patentamt
European Patent Office
Office européen des brevets

(11) Veröffentlichungsnummer: **0 219 654 B1**

# (12) EUROPÄISCHE PATENTSCHRIFT

(45) Veröffentlichungstag der Patentschrift: **08.08.90**

(51) Int. Cl.[5]: **C07C 47/14**, C07C 45/51, C07C 43/313, C07C 41/48

(21) Anmeldenummer: **86111811.5**

(22) Anmeldetag: **26.08.86**

## (54) Verfahren zur Herstellung von 4-Chlorbutanalen.

(30) Priorität: **24.10.85 DE 3537815**

(43) Veröffentlichungstag der Anmeldung: **29.04.87 Patentblatt 87/18**

(45) Bekanntmachung des Hinweises auf die Patenterteilung: **08.08.90 Patentblatt 90/32**

(84) Benannte Vertragsstaaten: **AT BE CH DE FR GB IT LI NL SE**

(56) Entgegenhaltungen:
**FR-A- 2 434 166**
**US-A- 3 686 215**

**TETRAHEDRON LETTERS, Band 26, Nr. 8, 1985, Seiten 961-964, Pergamon Press Ltd, GB; F. VANMIDDLESWORTH et al.: "Bifunctional chiral synthons via biochemical methods. VI. C5 isoprenoid units"**

(73) Patentinhaber: **Degussa Aktiengesellschaft, Weissfrauenstrasse 9, D-6000 Frankfurt am Main 1(DE)**

(72) Erfinder: **Andrade, Juan, Dr., 545 Barnett Place, Ridgewood, NJ 07450(US)**
Erfinder: **Prescher, Günter, Dr., Liesingstrasse 2, D-6450 Hanau 9(DE)**
Erfinder: **Köhler, Klaus, Kettelerstrasse 37, D-6452 Hainburg(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 219 654 B1

## Beschreibung

Die Erfindung betrifft ein Verfahren zur Herstellung von 4-Chlor-butanalen der Formel

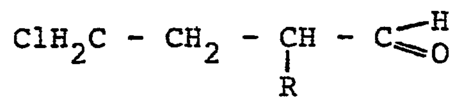 (I),

in der R Wasserstoff oder einen geradkettigen oder verzweigten Alkylrest mit I bis 4 Kohlenstoffatomen bedeutet. Die 4-Chlor-butanale der Formel (I) können mit gegebenenfalls substituierter Salicylhydroxamsäure zu 2,3,4,4a-Tetrahydro-I0H-I,2-oxazino [3,2-b]-[I,3]-benzoxazin-I0-onen kondensiert werden (vgl. Arzneim.-Forsch. Drug Res. 27 (I), Nr. 4 (I977), 760). (S)-4-Chlor-2-methyl-butanal ist ferner ein wichtiges Zwischenprodukt für die Herstellung von synthetischem Vitamin E (vgl. DE-OS 27 20 775).

Es ist bereits bekannt, 4-Chlor-butanal durch Hydrierung von 4-Chlor-buttersäurechlorid in Gegenwart eines vergifteten Edelmetallkatalysators (J. Am. Chem. Soc. 73, 1365 (1951)) oder in Gegenwart eines Legierungskatalysators (DE-OS 2 506 157) herzustellen. Die Ausbeute an 4-Chlor-butanal ist jedoch bei diesen bekannten Verfahren relativ schlecht.

Aus der FR-A 2 434 166 ist es ferner bekannt, 1-Brom-4-formyl-hexan durch Umsetzung von 4-Dimethoxymethyl-1-hexanol mit Tetrabromkohlenstoff in Gegenwart von Triphenylphosphin und anschließende saure Hydrolyse des gebildeten 1-Brom-4-dimethoxymethylhexans herzustellen.

Aus der US-A 3 686 215 ist es weiter bekannt, 3-Formylpropylbromid durch Umsetzung von 4,4-Dimethoxybutanol mit Tetrabromkohlenstoff in Gegenwart von Triphenylphosphin und anschließende saure Hydrolyse des gebildeten 4,4-Dimethoxybutylbromids herzustellen.

Aus "Advanced Organic Chemistry, Jerry March, 3. Auflage, John Wiley and Sons, Seiten 382/383, ist es schließlich ganz allgemein bekannt, Alkohole durch Umsetzung mit einem Gemisch aus Triphenylphosphin und Tetrachlorkohlenstoff in die entsprechenden Alkylchloride umzuwandeln.

Das erfindungsgemäße Verfahren ist nun dadurch gekennzeichnet, daß man

a) ein 1,1-Dimethoxy-4-hydroxy-butan der Formel

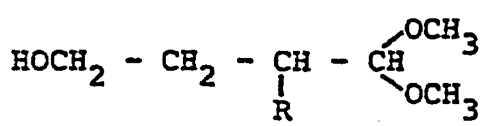 (II),

in der R die bereits angegebene Bedeutung hat, bei einer Temperatur zwischen –20 und +80°C in Gegenwart der 0,1- bis 20-fachen molaren Menge an Triphenylphosphin, eines tertiären Amins mit einem Siedepunkt unter 100°C/1 bar und eines tertiären Amins mit einem Siedepunkt über 200°C/1 bar mit der 1,0- bis 10-fachen molaren Menge an Tetrachlorkohlenstoff umsetzt und

b) das in der Reaktionsstufe a) gebildete 1,1-Dimethoxy-4-chlor-butan der Formel

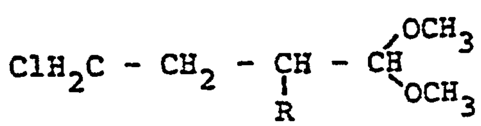 (III),

in der R wieder die bereits angegebene Bedeutung hat, in an sich bekannter Weise in saurem Medium hydrolysiert.

Durch diese Reaktionsfolge können 4-Chlor-butanale der Formel (I) auf einfache Weise und in guter Ausbeute hergestellt werden.

Die in der Reaktionsstufe a) als Ausgangsmaterial dienenden 1,1-Dimethoxy-4-hydroxy-butane der Formel (II) können leicht durch Hydroformylierung von gegebenenfalls in 2-Stellung substituiertem Acroleindimethylacetal und anschließende Hydrierung der Formylgruppe hergestellt werden.

Die Reaktionsstufe a) wird vorzugsweise bei einer Temperatur zwischen +20 und +70°C durchgeführt. Das Triphenylphosphin wird vorzugsweise in der 1- bis 2-fachen molaren Menge, der Tetrachlorkohlenstoff vorzugsweise in der 1- bis 10-fachen molaren Menge eingesetzt. Zweckmäßigerweise wird so verfahren, daß man das 1,1-Dimethoxy-4-hydroxy-butan der Formel (II) langsam zu einer Lösung des Triphenylphosphins in dem Tetrachlorkohlenstoff zutropft, der zusätzlich noch ein tertiäres Amin mit einem Siedepunkt unter 100°C/1 bar und ein tertiäres Amin mit einem Siedepunkt über 200°C/1 bar enthält.

Das niedriger siedende tertiäre Amin wird zweckmäßig in einer Menge zwischen 0,001 und 0,1 Gewichtsteilen, das höher siedende tertiäre Amin zweckmäßig in einer Menge zwischen 0,05 und 0,7 Gewichtsteilen angewandt, jeweils pro Gewichtsteil des eingesetzten 1,1-Dimethoxy-4-hydroxy-butans der

Formel (II). Geeignete niedrig siedende tertiäre Amine sind beispielsweise das Methyldiethylamin und das bevorzugte Triethylamin, geeignete höher siedende tertiäre Amine Tri-n-butylamin, N-Methylindol, Isochinolin oder das bevorzugte Chinolin.

Während der Umsetzung bildet sich ein weißer Niederschlag von Triphenylphosphinoxid. Nach beendeter Zugabe des 1,1-Dimethoxy-4-hydroxy-butans der Formel (II) wird zweckmäßigerweise das Reaktionsgemisch noch weitere 2 bis 3 Stunden gerührt und dann bei Raumtemperatur filtriert. Das Filtrat wird auf ein kleineres Volumen eingeengt und das gebildete 1,1-Dimethoxy-4-chlor-butan der Formel (III) wird unter vermindertem Druck fraktioniert destilliert.

Die Reaktionsstufe b) wird zweckmäßigerweise bei einer Temperatur zwischen 20 und 60 °C durchgeführt. Sie erfolgt in an sich bekannter Weise in saurem Medium, das heißt in Gegenwart eines stark sauren Ionenaustauschers oder vorzugsweise einer Mineralsäure, insbesondere verdünnter Schwefelsäure. Man kann beispielsweise so vorgehen, daß man das l,l-Dimethoxy-4-chlor-butan der Formel (III) unter kräftigem Rühren mit überschüssiger verdünnter wäßriger Schwefelsäure versetzt. Nach 2 bis 3 Stunden wird dann das Reaktionsgemisch mit Methylenchlorid extrahiert. Die vereinigten Methylenchloridphasen werden mit Natriumcarbonatlösung und mit Wasser ausgewaschen und über Magnesiumsulfat getrocknet. Nach dem Abfiltrieren des Magnesiumsulfats wird das Methylenchlorid abgedampft und der Rückstand wird unter vermindertem Druck fraktioniert destilliert.

Die Erfindung wird durch die nachfolgenden Beispiele näher erläutert:

Beispiel l:

l29 g (0,96 Mol) l,l-Dimethoxy-4-hydroxy-butan wurden bei 20 °C zu einer Lösung von 3l5 g (l,2 Mol) Triphenylphosphin, 67,2 g Chinolin und 2,9 g Triethylamin in 362 ml Tetrachlorkohlenstoff unter Kühlung langsam zugetropft. Nach beendeter Zugabe stieg die Temperatur bei der Nachreaktion auf ca. 60 °C, wobei Triphenylphosphinoxid allmählich als weißer Niederschlag ausfiel. Das Reaktionsgemisch wurde noch zwei Stunden gerührt, auf 20 °C abgekühlt und filtriert. Das Filtrat wurde dann unter vermindertem Druck bei l8 mbar destilliert. Das gewünschte l,l-Dimethoxy-4-chlor-butan ging bei 68 °C über. Die Ausbeute, bezogen auf eingesetztes l,l-Dimethoxy-4-hydroxy-butan,betrug l23 g oder 84 % der Theorie.

Dieses wurde bei 50 °C zu l Liter 0,2 N Schwefelsäure zugetropft und noch zwei Stunden nachgerührt. Nach Abkühlung auf 20 °C wurde die organische Phase abgetrennt, die wäßrige Phase 3 mal mit je 200 ml Methylenchlorid extrahiert, die vereinigten organischen Phasen mit Natriumcarbonat-Lösung neutral gewaschen, über Magnesiumsulfat getrocknet und bei 50 mbar destilliert. Das gewünschte 4-Chlor-butanal ging bei 74 °C über. Die Ausbeute betrug 80 g oder 93 % der Theorie.

Beispiel 2:

74 g (0,5 Mol) l,l-Dimethoxy-2-methyl-4-hydroxy-butan wurden bei 40 °C zu einer Lösung von l57,5 g (0,6 Mol) Triphenylphosphin, 34 g Chinolin und l,5 g Triethylamin in l8l ml Tetrachlorkohlenstoff zugetropft, wobei allmählich Triphenylphosphinoxid ausfiel. Das Reaktionsgemisch wurde noch zwei Stunden bei 40 °C gerührt und danach wurde wie in Beispiel l verfahren. Die Ausbeute an l,l-Dimethoxy-2-methyl-4-chlor-butan, bezogen auf eingesetztes l,l-Dimethoxy-2-methyl-4-hydroxy-butan betrug 64,9 g (78 % der Theorie) mit einem Siedepunkt von 73 °C bei 20 mbar. Dieses wurde dann weiter wie im Beispiel l zu 4-Chlor-2-methyl-butanal hydrolysiert. Ausbeute 44,4 g (94,7 % der Theorie) mit einem Siedepunkt von 76 bis 78 °C bei 40 mbar.

Beispiel 3:

Es wurde wie im Beispiel l verfahren, jedoch wurden 33,5 g (0,25 Mol) l,l-Dimethoxy-4-hydroxy-butan, 78,7 g (0,3 Mol) Triphenylphosphin, 3,4 g Chinolin, 3,4 g Triethylamin und 240 ml Tetrachlorkohlenstoff eingesetzt. Die Ausbeute an 4-Chlor-butanal, bezogen auf eingesetztes l,l-Dimethoxy-4-hydroxy-butan betrug 2l g oder 79 % der Theorie.

Beispiel 4:

Es wurde wie im Beispiel 2 verfahren, jedoch wurden 370 g (2,5 Mol) l,l-Dimethoxy-2-methyl-4-hydroxy-butan, 787 g (3 Mol) Triphenylphosphin, 220 g Chinolin und 33 g Triethylamin in 2 Liter Tetrachlorkohlenstoff eingesetzt. Die Ausbeute an 4-Chlor-2-methyl-butanal, bezogen auf eingesetztes l,l-Dimethoxy-2-methyl-4-hydroxy-butan, betrug 244 g oder 8l % der Theorie.

**Patentansprüche**

1. Verfahren zur Herstellung von 4-Chlor-butanalen der Formel

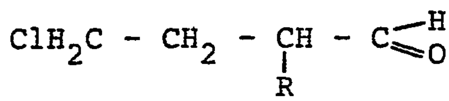

(I),

in der R Wasserstoff oder einen geradkettigen oder verzweigten Alkylrest mit 1 bis 4 Kohlenstoffatomen bedeutet, dadurch gekennzeichnet, daß man

a) ein 1,1-Dimethoxy-4-hydroxy-butan der Formel

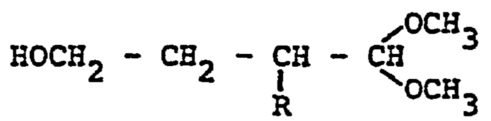

(II),

in der R die bereits angegebene Bedeutung hat, bei einer Temperatur zwischen –20 und +80°C in Gegenwart der 0,1- bis 20-fachen molaren Menge an Triphenylphosphin, eines tertiären Amins mit einem Siedepunkt unter 100°C/1 bar und eines tertiären Amins mit einem Siedepunkt über 200°C/1 bar mit der 1,0- bis 10-fachen molaren Menge an Tetrachlorkohlenstoff umsetzt und

b) das in der Reaktionsstufe a) gebildete 1,1-Dimethoxy-4-chlor-butan der Formel

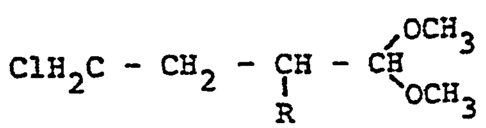

(III),

in der R wieder die bereits angegebene Bedeutung hat, in an sich bekannter Weise in saurem Medium hydrolysiert.

2. Verfahren nach Anspruch 1, dadurch gekennzeichnet, daß man das niedriger siedende tertiäre Amin in einer Menge zwischen 0,001 und 0,1 Gewichtsteilen pro Gewichtsteil eingesetzten 1,1-Dimethoxy-4-hydroxy-butans der Formel (II) einsetzt.

3. Verfahren nach Anspruch 1 oder 2, dadurch gekennzeichnet, daß man das höher siedende tertiäre Amin in einer Menge zwischen 0,05 und 0,7 Gewichtsteilen pro Gewichtsteil eingesetzten 1,1-Dimethoxy-4-hydroxy-butans der Formel (II) einsetzt.

4. Verfahren nach einem der Ansprüche 1 bis 3, dadurch gekennzeichnet, daß man als niedriger siedendes tertiäres Amin Triethylamin und als höher siedendes tertiäres Amin Chinolin verwendet.

**Claims**

1. Process for the preparation of 4-chloro-butanals corresponding to the following formula

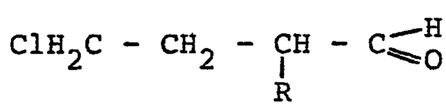

(I)

wherein R denotes hydrogen or a straight chain or branched alkyl group having 1 to 4 carbon atoms, characterised in that

a) a 1,1-dimethoxy-4-hydroxy-butane corresponding to the following formula

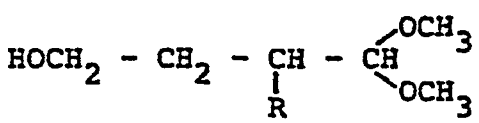

(II)

wherein R has the meaning indicated above is reacted at a temperature from –20°C to +80°C with from 1.0 to 10 times the molar quantity of carbon tetrachloride in the presence of from 0.1 to 20 times the molar quantity of triphenyl phosphine, a tertiary amine having a boiling point below 100°C/1 bar and a tertiary amine having a boiling point above 200°C/1 bar and

b) the 1,1-dimethoxy-4-chloro-butane formed in reaction stage a) corresponding to the following formula

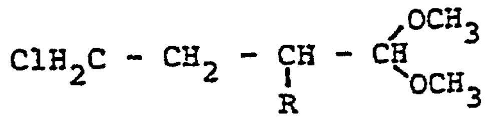 (III)

wherein R again has the meaning already indicated, is hydrolysed in an acid medium in known manner.

2. Process according to Claim 1, characterised in that the lower boiling tertiary amine is used in a quantity of from 0.001 to 0.1 parts by weight per part by weight of 1,1-dimethoxy-4-hydroxy-butane of formula (II) put into the process.

3. Process according to Claim 1 or Claim 2, characterised in that the higher boiling tertiary amine is used in a quantity of from 0.05 to 0.7 parts by weight per part by weight of 1,1-dimethoxy-4-hydroxy-butane of formula (II) put into the process.

4. Process according to one of the Claims 1 to 3, characterised in that the lower boiling tertiary amine used is triethylamine and the higher boiling tertiary amine used is quinoline.

**Revendications**

1. Procédé d'obtention de 4-chlorobutanals de formule:

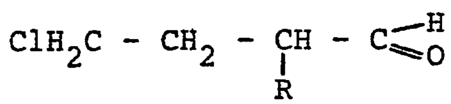 (I)

dans laquelle R signifie de l'hydrogène ou un radical alcoyle linéaire ou ramifié ayant de 1 à 4 atomes de carbone, caractérisé en ce que

a) un 1,1-diméthoxy-4-hydroxybutane de formule:

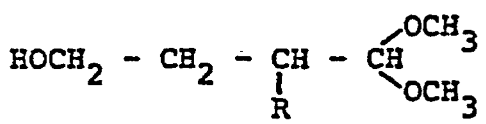 (II)

dans laquelle R possède les significations déjà mentionnées,
est mis à réagir à une température comprise entre −20 et +80°C en présence d'une quantité 0,1 à 20 fois molaire de triphénylphosphine, d'une amine tertiaire ayant un point d'ébullition en-dessous de 100°C/1 bar et d'une amine tertiaire ayant un point d'ébullition au-dessus de 200°C/1 bar avec une quantité 1,0 à 10 fois molaire de tétrachlorure de carbone et,

b) le 1,1-diméthoxy-4-chlorobutane formé à l'étape réactionnelle a) de formule:

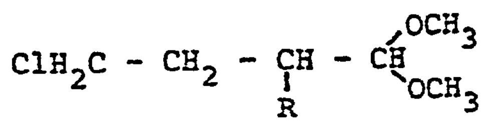 (III)

dans laquelle R à nouveau a la signification déjà mentionnée,
est hydrolysé d'une manière connue en soi en milieu acide.

2. Procédé selon la revendication 1, caractérisé en ce que l'on met en œuvre l'amine tertiaire à plus bas point d'ébullition en quantité allant de 0,001 à 0,1 partie en poids par partie en poids de 1,1-diméthoxy-4-hydroxybutane de formule (II) mis en jeu.

3. Procédé selon la revendication 1 ou la revendication 2, caractérisé en ce que l'on met en œuvre l'amine tertiaire à plus haut point d'ébullition en quantité allant de 0,05 à 0,7 partie en poids par partie en poids de 1,1-diméthoxy-4-hydrobutane de formule (II) mis en jeu.

4. Procédé selon l'une des revendications 1 à 3, caractérisé en ce que l'on utilise en tant qu'amine tertiaire à plus bas point d'ébullition, de la triéthylamine et en tant qu'amine tertiaire à plus haut point d'ébullition, de la quinoléine.